# EUROPÄISCHE PATENTSCHRIFT

(11) **EP 2 934 343 B1**
(45) Veröffentlichungstag und Bekanntmachung des Hinweises auf die Patenterteilung: **05.05.2021**
(21) Anmeldenummer: 13815487.7
(22) Anmeldetag: 19.12.2013
(51) Int. Cl.: A61B 17/02, A61B 17/00, A61B 17/30

(54) **CHIRURGISCHES POSITIONIERINSTRUMENT ZUM ABSTÜTZEN UND HALTEN VON ORGANEN**
SURGICAL POSITIONING INSTRUMENT FOR SUPPORTING AND HOLDING ORGANS
INSTRUMENT DE POSITIONNEMENT CHIRURGICAL POUR SOUTENIR ET RETENIR DES ORGANES

(30) Priorität: 20.12.2012 DE 102012112787
(43) Veröffentlichungstag der Anmeldung: 28.10.2015
(73) Patentinhaber: Aesculap AG, 78532 Tuttlingen (DE)
(72) Erfinder: GALAJDA, Zoltan Prof. Dr., 4032 Debrecen (HU); SEYFRIED, Dominik, 78126 Königsfeld (DE); LUTZE, Theodor, 78582 Balgheim (DE); WEISSHAUPT, Dieter, 78194 Immendingen (DE); BECK, Thomas, 78591 Durchhausen (DE); VERSE, Sonja, 78604 Rietheim-Weilheim (DE); VOGTHERR, Robert, 78532 Tuttlingen (DE); ELISCH, Andreas, 78713 Schramberg (DE); MORALES, Pedro, 78532 Tuttlingen (DE); DZEMALI, Omer Dr. med., 8063 Zürich (CH); PATONAY, Lajos Dr., 1141 Budapest (HU)
(74) Vertreter: Winter, Brandl - Partnerschaft mbB
(86) Internationale Anmeldenummer: PCT/EP2013/077482
(87) Internationale Veröffentlichungsnummer: WO 2014/096252

(56) Entgegenhaltungen:
- WO-A2-99/20321
- US-A1- 2002 095 139
- US-A1- 2004 138 522

## Beschreibung

Die Erfindung betrifft ein chirurgisches Positionierinstrument zum Abstützen und Halten von Organen gemäß dem Oberbegriff des Anspruchs 1.

### Technischer Hintergrund

Bei Operationen an Organen wie beispielsweise am Herzen ist es notwendig, dass das Organ zumindest temporär positioniert wird. Das ist zum einen wichtig, damit dem Operateur die Operationsstelle des Organs gut zugänglich ist. Außerdem dürfen sich gerade sensible Organe, wie es insbesondere beim Herzen der Fall ist, während des Eingriffs nicht bewegen. Daher sind verschiedenste Technologien und Vorrichtungen zum Positionieren von Organen während einer Operation bekannt.

So können Organe durch die menschliche Hand alleine oder durch Zuhilfenahme von Instrumenten wie Wundhaken gestützt bzw. gehalten werden. Von Nachteil ist, dass auf diese Weise eine ungleichmäßige Druckverteilung auf das Organ ausgeübt wird. Ferner kann eine Position nicht dauerhaft fixiert werden, insbesondere weil die menschliche Hand vergleichsweise schnell ermüdet und zu Zucken beginnt. Zudem stellt sie eine starke Sichtbehinderung dar.

Werden OP-Tücher untergelegt, ist zumindest die Sicht des Operateurs frei. Jedoch ist auch hier weder eine komfortable noch eine dauerhafte Positionierung sichergestellt, da die OP-Tücher fortwährend Feuchtigkeit aufnehmen und somit an Stabilität verlieren. Außerdem ist auch das Einlegen von OP-Tüchern, bis die gewünschte Position erreicht ist, zeitintensiv.

Aus EP 1 002 498 A1, US 5 735 791 A und US 6 036 640 A sind aufblasbare Positionierungskissen, und aus US 4 637 377 A und EP 1 028 656 B1 sind aufblasbare Keile bekannt, wobei die zuletzt genannte Druckschrift mit einer Vielzahl von separaten Fluidkammern arbeitet. Diese Gegenstände basieren alle darauf, dass sie dem relevanten Organ untergeschoben und anschließend aufgeblasen bzw. mit Fluid gefüllt werden.

Nachteilig an diesen Systemen ist, dass sie wenig Rücksicht auf die anatomischen Gegebenheiten der Organe nehmen und folglich nicht komfortabel sind. Die nicht angepasste Form führt ferner dazu, dass die Organe insbesondere beim Keil eingeklemmt werden müssen und so lokale Druckstellen am Organ entstehen, was zu einer verstärkten Hämatombildung führt. Ferner wird dadurch die Hämodynamik des Herzen eingeschränkt.

Zusätzlich sind aus US 6 899 670 B2 sowie US 7 438 860 B2 vakuumunterstützte Positionierungssysteme bekannt. Diese Systeme bestehen im Wesentlichen aus einem im Vorfeld zu montierenden Gerüst mit einem Arm bzw. Gliederarm, über das ein Saugnapf am Gerüst montiert und am Organ positioniert wird. Ferner erzeugt eine Vakuumpumpe einen relativen Unterdruck, der mittels einer aufwendigen Drucküberwachungsapparatur an den Saugnapf weitergeleitet wird. Dieser saugt das Organ an und positioniert es auf diese Weise. Nachteiligerweise sind diese Systeme sehr sperrig. Außerdem bedingen sie einen hohen Montageaufwand, bevor mit der Operation nach Positionierung des Organs begonnen werden kann. Zudem sind sie kostenintensiv.

Die Patentschrift US 5 453 078 A offenbart eine keilförmig ausgebildete Schwammvorrichtung mit einem steifen und saugfähigen Schwammkörper. Der Schwamm wird vorzugsweise dazu eingesetzt, um entweder das zu operierende Organ zugänglich zu machen, indem im Weg befindliche Organe bei Seite geschoben werden, oder um das zu operierende Organ in das Operationsfeld zu bringen. Im Einsatz wird der keilförmige Schwamm zwischen den Organen eingeklemmt, so dass er sich mit steigender Feuchtigkeitsaufnahme um ein Mehrfaches seiner Ausgangsgröße entsprechend ausdehnt und die Organe einklemmt.

Nachteilig daran ist, dass der anfangs steife Schwamm eine ungleichmäßige und meist im Wesentlichen punktförmige Druckverteilung auf das Organ ausübt, was nicht komfortabel ist und Hämatome verursacht. Zudem schränkt eine nicht komfortable Lagerung die Hämodynamik des Herzen ein.

Hinzu kommt, dass der Keil durch Ausprobieren in eine günstige und stabile Position gebracht wird, so dass sowohl das Organ auf die gewünschte Weise positioniert, als auch der Keil sicher eingeklemmt ist. Dieser Vorgang ist zeitintensiv und es kann selbst dann nicht ausgeschlossen werden, dass sich der Keil jeder Zeit wieder löst.

Zudem ändert sich im Laufe des kontinuierlichen Vollsaugens des Schwamms dementsprechend ständig der Positionierungszustand. Dieser geht immer weiter von einem verkeilten Zustand in einen das Organ aufnehmenden und teilweise umschließenden Zustand über. Da sich hierbei die gesamte Druckverteilung sowohl zwischen Organ und Schwamm, als auch zwischen Schwamm und Abstützfläche ändert, verändert sich dabei auch zwangsweise die Positionierung des Organs. Demnach geht mit zunehmender Feuchtigkeitsaufnahme eine sinkende Stabilität der Positionierung einher.

Demgegenüber liegt der Erfindung die Aufgabe zu Grunde, ein hinsichtlich der Organgröße möglichst universell einsetzbares Positionierinstrument zum zuverlässigen Aufnehmen und Halten von Organen bereitzustellen, wobei die Funktion des Organs möglichst minimal negativ beeinflusst wird.

Diese Aufgabe wird gelöst durch ein chirurgisches Positionierinstrument mit den Merkmalen des Anspruchs 1. Vorteilhafte Weiterbildungen des chirurgischen Positionierinstruments sind in den Ansprüchen 2 bis 14 beschrieben.

Das erfindungsgemäße chirurgische Positionierinstrument (nachfolgend Instrument genannt) zum Abstützen und Halten von Organen weist gemäß einem Aspekt der Erfindung an einer Seitenfläche einen konkaven Lagerabschnitt auf, über welchen ein Organ aufnehmbar ist. An seiner zum Lagerabschnitt rückwärtigen Seitenfläche weist das Instrument einen vorzugsweise konvexen Stützabschnitt auf, über welchen es an einer Umgebung des Organs ohne manuelle Hilfe abstützbar ist. Eine Oberfläche des konkaven Lagerabschnitts ist zusätzlich zu der konkaven Grundform im Wesentlichen an eine Außenfläche, insbesondere an eine Hülle bzw. Hüllfläche des zu positionierenden Organs derart angepasst und (unabhängig vom Kernmaterial des Instruments/Stützkörpers) formstabil nachempfunden, dass es durch die möglichst ideale Passform das Organ mit einer über die Lagerfläche gleichmäßig verteilten Druckverteilung stützt und dauerhaft hält.

Ein Vorteil ist weiterhin, dass das formstabile Instrument schnell positioniert werden kann, ohne eine zusätzliche Fixierung mittels Tücherunterlagen und Schlaufen oder Spannschnüren am Umgebungsgewebe zu erfordern. Von besonderem Vorteil ist die ideale Passform dahingehend, als dass sie verhindert, dass wichtige Gefäße und / oder Gefäßstrukturen an den Organen abgeklemmt werden. Gerade beim Herzen ist dieser Vorteil von Bedeutung, da somit die Hämodynamik des Herzen nicht eingeschränkt wird.

Ein vorteilhafter Effekt des angepassten und formstabilen Lagerabschnitts ist, dass der Einsetzaufwand bzw. Positionierungsaufwand insbesondere zu Beginn der Operation, bis mit der eigentlichen Operation am betroffenen Organ begonnen werden kann, minimiert wird. Erreicht wird dies insbesondere dadurch, indem das Positionierinstrument/der Stützkörper an die Außenseite des Zielorgans, beispielsweise des Herzens schalenförmig angelegt wird, um dann aufgrund des rückseitigen konvexen Stützfläche zwischen das Ziel-Organ und das Umgebungsgewebe zu gleiten und dabei den Stützdruck in das Umgebungsgewebe zunehmend abzuleiten und damit das Ziel-Organ festzulegen.

In einer besonders bevorzugten Weiterbildung kann das Instrument ein im Wesentlichen keilförmig, insbesondere Faustkeil-förmig ausgebildetes Heart Pad / Stützkörper sein, welcher in die Perikardhöhle des Herzens einführbar ist. Die Lagerflächenoberfläche des Heart Pads / Stützkörpers ist zumindest abschnittsweise möglichst nahe einer Oberflächenkontur des Herzen nachgebildet.

Die in Einführrichtung sich verjüngende Keilform / Faustkeilform des Heart Pads / Stützkörpers ermöglicht ein einfaches Einführen in die Perikardhöhle. Dabei wird das Herz von dem konkaven sowie zusätzlich an die Organform angeglichenen Lagerabschnitt des Heart Pads so aufgenommen, dass das Epikard und der Lagerabschnitt miteinander in Kontakt stehen, während sich das Heart Pad über den rückwärtigen, vorzugsweise konvex ausgebildeten Stützabschnitt am Perikard abstützt.

Die Oberfläche des Lagerabschnitts ist zumindest in dem Bereich, an welchem diese mit dem Herzen in Kontakt steht, der Oberflächenkontur des Herzen nachgebildet. Mit der als negative Form des Herzen ausgebildeten Lagerfläche kann das Heart Pad somit das Herz ideal Abstützen und Halten.

Die ideale Passform des Heart Pads zusammen mit der Keilform / Faustkeilform in der Einführrichtung sowie in einer dazu senkrechten Tiefenrichtung des Heart Pads hat den besonderen Vorteil, dass das Heart Pad ohne zusätzliche Fixierungen (beispielsweise über ein chirurgisches Instrument) in der Perikardhöhle in der gewünschten Lage verbleibt. Das wird dadurch erreicht, dass durch das Einführen des Heart Pads Epikard und Perikard gespannt werden und auf diese Weise das Herz positionieren.

Ein weiterer Vorteil liegt darin, dass keine sperrigen und komplexen Apparaturen oder manuelle Halteinstrumente zum Halten/Fixieren des Heart Pads erforderlich sind, wodurch ferner die freie Sicht eines Operateurs nicht behindert wird.

Die einfache Bauweise bringt einen deutlichen Kostenvorteil sowie einen reduzierten Montageaufwand mit sich. Zusätzlich kann durch den Wegfall der Montageapparatur kostbare Zeit hinsichtlich der Patientengesundheit wie auch kostenintensive Zeit hinsichtlich der OP-Zeit dadurch gespart werden, da das Herz schneller positioniert ist.

Um eine Operationsstelle ruhigzustellen, kann das Heart Pad in einer bevorzugten Ausführungsform eine integrierte Sensor- / Aktuatorkombination aufweisen. Hierbei erfasst der Sensor eine Herzbewegung und ein Aktuator erzeugt basierend auf einem Erfassungsergebnis des Sensors einen Impuls, um eine Relativbewegung zwischen dem Herzen und dem Heart Pad zu minimieren. Vorteil des Bewegungsausgleichs ist, dass das Herz ungehindert weiterschlagen kann und dem Operateur zugleich eine weitestgehend ruhige Operationsstelle vorliegt. Diese Ausführungsform schränkt zudem nicht die Hämodynamik des Herzen ein. Alternativ oder ergänzend zur Sensor- / Aktuatorkombination kann das Material des Heart Pads zumindest an Teilbereichen besonders gute dämpfende Materialeigenschaften aufweisen, um die Bewegungsenergie des Herzen zu absorbieren.

Nach einer besonders bevorzugten Weiterbildung kann das Heart Pad / der Stützkörper entsprechend den anatomischen Gegebenheiten des Organs wenigstens eine zusätzliche / weitere Aussparung aufweisen, welche eine eigene Auflagerfläche ausbildet.

Derart gezielt eingebrachte, eigene Auflagerflächen jeweils ausbildende Aussparungen haben den Vorteil, dass von dem Organ vorspringende Bereiche wie Blutgefäße oder dergleichen von den Aussparungen großflächig (nicht schafkantig) aufgenommen werden können, derart, dass das Organ im Übrigen von der Lagerfläche ebenfalls großflächig abgestützt und gehalten werden kann. Somit entstehen weder Hämatome aufgrund von im Wesentlichen punktförmiger Belastung, noch werden wichtige Gefäße und / oder Gefäßstrukturen abgeklemmt oder auf sonstige Weise negativ beeinträchtigt. Zusätzlich wird damit die Funktion des Organs, im Fall des Herzen die Hämodynamik, nicht eingeschränkt.

Die Aussparungen können alleine durch ihre markante Ausprägung ein fehlerhaftes Anbringen des Instruments verhindern. D.h. deren Ausrichtung sowie Positionierung am Stützkörper bezüglich der Organ-Lagerfläche ist entsprechend dem abzustützenden Zielorgan gewählt, sodass ein falsches Einsetzen des Stützkörpers ausgeschlossen ist. Zusätzlich oder alternativ kann das Instrument mit vorzugsweise dauerhaft angebrachten Hinweisen oder Kennzeichnungen versehen sein, die einen Verwendungszweck der jeweiligen Ausnehmung erläutern, so dass das Instrument korrekt verwendet werden kann.

Um das Instrument im Betrieb auf einfache und schnelle Weise einführen und entnehmen zu können, kann es ferner einen vorzugsweise am proximalen Ende angeordneten, als Montage- und Entnahmehilfe ausgebildeten Greifabschnitt aufweisen. Dieser ist vorzugsweise in Form von zumindest einer Öse oder Schlaufe ausgebildet, an welchem das Instrument gegriffen bzw. aufgenommen werden kann.

Vorteilhafterweise ist der Abschnitt so ausgebildet, dass er mit Hilfe eines in jedem Siebkorb befindlichen Instruments aufgenommen und geführt werden kann. Somit kann die Handhabung des Instruments ohne zur Hilfenahme der Hand erfolgen. Das ist insbesondere von Vorteil, weil lediglich ein Schieben des Instruments mit der Hand bzw. mit einem Finger mittels eines einfachen Kontakts möglich ist. Jede andere Bewegung wie etwa ein Herausnehmen bzw. Ziehen sowie jede Rotation des Instruments bedingt, dass sie von der Hand mit mindestens zwei Fingern gegriffen wird. Dies benötigt vergleichsweise viel Platz am Organ, der meistens nicht gegeben ist.

Im Gegensatz dazu ermöglicht die Montage- und Entnahmehilfe, dass das Instrument/der Stützkörper an seinem proximalen Ende gegriffen und sowohl translatorisch als auch rotatorisch beliebig bewegt werden kann.

Alternativ oder ergänzend kann das Instrument insbesondere an proximalen Abschnitten mit zumindest einer als Schlaufe, Lasche oder dergleichen ausgebildeten Hilfs- und / oder Haltevorrichtung versehen sein, woran vorzugsweise Hilfsnähte anbringbar sind. Somit sind weder weitere Halterung zum Gegenhalten der während einer Operation erforderlichen Nähte erforderlich, noch müssen die Nähte an das umgebende Gewebe angebracht werden und dieses dadurch verletzen.

Die eben beschriebenen Fixierungshilfen können an einer oder mehreren Flächen des Instruments / Stützkörpers wie beispielsweise einem Heart Pad vorgesehen sein. So kann es bei kritischen Operationen notwendig sein, das erfindungsgemäße Instrument zusätzlich mit Haltenähten zu fixieren. In diesem Fall können Fixierungshilfen auch an distalen Flächen des Instruments vorgesehen sein, in die zunächst ein Fixierungsfaden eingefädelt wird, welcher nach dem Einsetzen des Instruments in die endgültige Position zur Haltenaht komplettiert wird.

Um Flüssigkeiten aus dem Operationsbereich absaugen zu können, kann das Instrument ferner eine Abführvorrichtung mit zumindest einem als Ausleitekanal und / oder Luftnachführkanal ausgebildeten Kanal aufweisen. Das Instrument kann insbesondere derart ausgebildet sein, dass in einen Kanal, dessen Querschnitt vorzugsweise eine Form eines Vielecks aufweist, ein weiterer Kanal mit einer davon verschiedenen Querschnittsform eingeführt ist. Auf diese Weise kann der eine Teil des Gesamtquerschnitts zum Abführen einer Flüssigkeit dienen, während der andere Teil zum Luftnachführen dient.

Die Abführvorrichtung dient zum Abführen von Flüssigkeiten. Insbesondere in der Perikardhöhle, von der das Heart Pad umgeben ist, befindet sich ein Perikarderguss, der mittels eines Kanals abgeführt werden kann. Vorzugsweise ist ein distales Ende des Kanals in Vertikalrichtung etwa an einer im eingeführten Zustand tiefsten Stelle des Heart Pads angeordnet und ein proximales Ende des Kanals ist etwa an einer der tiefsten Stelle entgegengesetzten Stelle des Instruments angeordnet. Das proximale Ende ist an eine Absaugeinrichtung wie beispielsweise eine Handabsaugpumpe, eine manuelle Absaugpumpe (Fußbetrieb) und / oder eine elektrisch betriebene Absaugpumpe bzw. einen Sauger anschließbar.

Die Abführvorrichtung kann in einer Weiterentwicklung anstelle eines Kanals zum Abführen von Fluiden sowie Zuführen von Luft alternativ oder ergänzend mit zumindest einem als Ausleitekanal ausgebildeten Abführkanal bzw. Saugkanal und zumindest einem als Luftzuführkanal ausgebildeten Luftkanal ausgebildet sein.

Vorzugsweise wird der Anschluss der Abführvorrichtung über ein einfaches und universelles Verbindungssystem wie dem Luer-System (Luer-Ansatz, -Lock, -Steck, - Slip) an eine Absaugeinrichtung realisiert.

Um ein Ansaugen von Gewebe an einer Saugöffnung des Abführkanals zu verhindern, kann ferner ein Entlüftungssystem integriert sein, derart, dass eine Aussparung im Heart Pad am distalen Endabschnitt der Abführvorrichtung vorgesehen ist, an der zumindest ein Luftkanal endet. Auf diese Weise kann Luft entsprechend dem Absaugvolumen nachströmen, um ein teilweises oder komplettes Verschließen bzw. Versperren der Saugöffnung zu verhindern.

Ferner kann der Bereich der Saugöffnung derart gestaltet sein, dass der Saugkanal an seinem distalen Ende in proximaler Richtung relativ zur Aussparung zurückgesetzt ist, so dass dessen distales Ende nicht aus dem Heart Pad vorspringt. Zusätzlich können Streben oder eine gitterähnliche Struktur im Bereich der Aussparung vorgesehen sein, welche einen Kontakt zwischen Gewebe und Saugkanal verhindern. Es ist zu beachten, dass der Kontakt nur dann verhindert werden kann, wenn zwischen dem distalen Saugkanalendabschnitt und der gitterähnlichen Struktur stets ein positiver Abstand aufrechterhalten bleibt.

Mit der beschriebenen Abführvorrichtung können folglich freie Flüssigkeiten wie flüssiges Blut, Erguss oder dergleichen unmittelbar und einfach abgeführt werden, ohne dass die eigentliche Operation unterbrochen werden muss, um einen Tupfer, Sauger etc. zu verwenden.

Alternativ oder ergänzend zur Abführvorrichtung kann das Heart Pad ganz oder teilweise aus saugfähigem Material bestehen. Dadurch wird die während der Operation auftretende Flüssigkeit direkt und quasi automatische aufgenommen, wobei die Abführvorrichtung unterstützend eingesetzt werden kann.

An dieser Stelle sei ausdrücklich darauf hingewiesen, dass die vorstehend genannte Abführvorrichtung nicht auf das Heart Pad beschränkt, sondern ebenso bei jedem anderen erfindungsgemäßen chirurgischen Positionierinstrument anwendbar ist. Auch muss die Abführvorrichtung nicht zwangsläufig körperlicher Bestandteil des chirurgischen Instruments sein sondern kann auch von außen in das chirurgische Instrument eingeführt werden.

Mit zunehmender Dauer der Operation kühlen Organe zunehmend aus. Um dem entgegenzuwirken, kann das Instrument zumindest am Lagerabschnitt ein wärmedämmendes Material aufweisen, um einen übermäßigen Wärmeverlust des Organs zu vermeiden. Ein Thermoelement kann zusätzlich oder alternativ vorzugsweise im Inneren des Instruments vorgesehen sein, um dem Organ bei Bedarf Wärme zuzuführen. Unter wärmedämmend soll vorliegend in bevorzugter Weise ein Material, (vorzugsweise ein elastisches Material) verstanden werden mit einer Wärmeleitfähigkeit von maximal 1W/(m*K).

In einer Weiterbildung können an einer gewünschten Position des Instruments Saugnäpfe und / oder Vakuumsegmente als Fixiervorrichtung vorgesehen sein. Ein relativer Unterdruck zwischen den Saugnäpfen und einer Umgebung wie beispielsweise dem Organ oder dergleichen kann beispielsweise durch Andrücken der Saugnäpfe erreicht wird. Es kann ebenso eine Vakuumquelle verwendet werden, die mit den Saugnäpfen derart verbunden ist, dass sie Luft zwischen den am Organ anliegenden Saugnäpfen und dem Organ absaugen kann, um auf diese Weise das Instrument am Organ zu fixieren.

Diese Fixiervorrichtung eignet sich zum einen dazu, das entsprechende Organ an das chirurgische Instrument zu fixieren. Zusätzlich oder alternativ kann die Fixiervorrichtung dazu verwendet werden, um das chirurgische Instrument lokal an der Umgebung zu fixieren.

Besonders vorteilhaft ist die Fixiervorrichtung daher bei einer Herzoperation. Hier wird das Heart Pad zum Fixieren und Positionieren des Herzen in die Perikardhöhle eingeführt. Am Lagerabschnitt befindliche Saugnäpfe fixieren das Herz über das Epikard und halten es an der gewünschte Position relativ zum Heart Pad, während am Stützabschnitt befindliche Saugnäpfe das Heart Pad an der Perikard fixieren und es somit an der gewünschten Position relativ zum Patienten halten. Auf diese Weise kann das Heart Pad in exakt die Position gebracht und dauerhaft gehalten werden, in der der Operateur freien Zugang zur gewünschte Stelle des Herzen hat.

Um zusätzlich ein Verrutschen zwischen dem Organ und dem Instrument bzw. dem umgebenden Gewebe zu verhindern, kann vorzugsweise die Oberfläche am Lagerabschnitt bzw. am Stützabschnitt mit einem Netz, einem Textilüberzug oder dergleichen versehen sein. Besonders von Vorteil ist diese Ausführungsform, wenn der Oberflächenüberzug zudem saugfähig ist, so dass ein möglicher Schmierfilm aufgesaugt wird. Dies ermöglicht zudem, dass Flüssigkeiten während der Operation direkt aufgenommen werden, so dass diese nicht abgesaugt und abgeführt werden müssen.

Um die Bewegungs- und die Positionierungsfähigkeit noch weiter zu verbessern, kann das Instrument eine Vielzahl von individuell befüllbaren Fluidkammern aufweisen. Das Volumen der jeweiligen Kammern kann von außen laufend individuell variiert werden. Durch ein Zusammenspiel der verschiedenen Kammern wird dem Operateur eine einfache und effektive Manipulationsmöglichkeit gegeben, um im Laufe der Operation die Position des Organs zu variieren sowie eine Feineinstellung vornehmen zu können. Insbesondere erweist sich ein System aus mehreren Kammern dahingehend als vorteilhaft, als dass weder der Operateur noch eine Assistenzperson unter Anweisung des Operateurs das Organ direkt mit den Händen verrücken und erneut fixieren müssen, sondern es bequem über ein Befüllen und / oder Entleeren der Fluidkammern durchführen können.

Die Anpassungsfähigkeit des Instruments an das Organ bzw. an die Stützfläche kann weiter verbessert werden, indem das Instrument einen weichen, vorzugsweise die äußere Hülle betreffenden und einen steifen, vorzugsweise den Kern betreffenden Abschnitt aufweist. Während der Kern eine tragende Aufgabe übernimmt, um dem Instrument die notwendige Formstabilität zu geben, dient die weiche Außenhaut dazu, um sich der Gegebenheiten ihrer Kontaktfläche anzupassen. Besonders vorteilhaft ist es, wenn eine viskoelastische Außenhaut verwendet wird.

Alternativ kann das Instrument eine Hülle aufweisen, deren Lagerabschnitt und / oder Stützabschnitt mit einem Granulat befüllt ist. Der Hülle kann beispielsweise über ein Ventil die darin enthaltene Luft entzogen werden. Folglich ermöglicht die Hülle eine bessere Anpassbarkeit einer Außenhaut des Instruments an den jeweiligen Patienten. Das wird dadurch erreicht, dass sich zunächst die anpassbare Außenhaut des Instruments der Form des Organs oder der stützenden Umgebung anpassen kann. Ist die optimale Form erreicht, wird ein relativer Unterdruck in dieser Hülle erzeugt, so dass die die einzelnen Granulatteilchen aneinander gedrückt werden und die Außenhaut steif und die aktuelle Positionierung gehalten wird. Auf diese Weise kann die Anpassbarkeit des Instruments noch weiter verbessert werden.

In einer besonders bevorzugten Weiterbildung kann das Instrument ein modular aufgebautes System darstellen. Dabei ist insbesondere ein zweiteiliges System sinnvoll, bei dem Lagerabschnitte und Stützabschnitten mit unterschiedlichen Abmaßen, Formen und Ausgestaltungen kombiniert werden können.

Denkbar ist ebenso ein als Luftkissen ausgebildetes Modul, welches in das Instrument integriert werden kann. Damit kann der Operateur während der Operation das Herz durch Befüllen und Entleeren des Luftkissens mit einem Fluid navigieren.

Damit kann das Instrument individuell an den jeweiligen Patienten angepasst werden, wodurch die Organe noch besser unterstützt und positioniert werden können und Nachteile wie Hämatome, eingeschränkte Hämodynamik des Herzen und dergleichen effektiver verhindert werden können.

Des Weiteren können Zusatzelemente wie Saugnäpfe und Vorrichtungsteile jeweils mit oder ohne Textilüberzug, Montagehilfe, Haltevorrichtung und dergleichen individuell zusammengesetzt werden, um ein optimal an den Patienten angepasstes Instrument zum Positionieren von Organen zu erhalten.

Weiterhin vorteilhaft daran ist, dass defekte oder verschlissene Teile ausgetauscht werden können, ohne das gesamte Instrument ersetzten zu müssen. Alternativ können zumindest manche der Teile als Wegwerfteile ausgebildet sein.

### Kurze Beschreibung der Zeichnungen

Im Folgenden werden bevorzugte Ausführungsbeispiele eines erfindungsgemäßen chirurgischen Instruments zum Abstützen und Halten von Organen unter Bezugnahme auf die beigefügten Zeichnungen näher erläutert.
Fig. 1 zeigt eine schematische, perspektivische Darstellung eines chirurgischen Instruments in einer Rückansicht gemäß einem ersten Ausführungsbeispiel der Erfindung;
Fig. 2 zeigt das chirurgische Instrument gemäß Fig. 1 in einer Vorderansicht;
Fig. 3 zeigt eine schematische, perspektivische Darstellung eines chirurgischen Instruments in einer Rückansicht gemäß einem zweiten Ausführungsbeispiel der Erfindung;
Fig. 4 zeigt eine schematische, perspektivische Darstellung eines chirurgischen Instruments in einer Rückansicht gemäß einem dritten Ausführungsbeispiel der Erfindung; und
Fig. 5 zeigt eine Detailansicht einer Aussparung in Fig. 4. Detaillierte Beschreibung der Zeichnungen

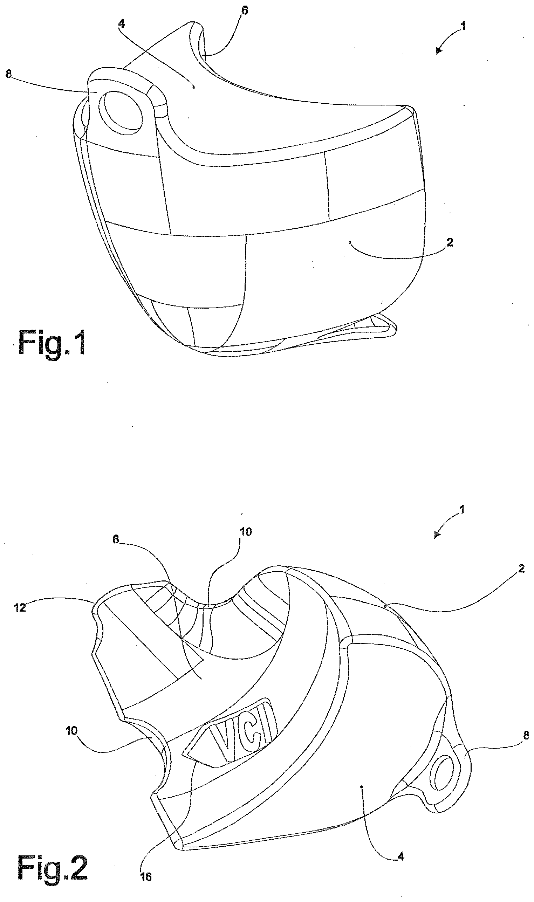

Fig. 1 zeigt in einer perspektivischen Ansicht beispielhaft ein Heart Pad 1 als ein chirurgisches Instrument zum Positionieren von Organen. Es sei angemerkt, dass die obere Seite in Fig. 1 einer in Einführrichtung Unterseite des Heart Pads 1 entspricht. Das Heart Pad 1 besteht im Wesentlichen aus drei Flächen. Eine mit dem Bezugszeichen 4 gekennzeichnete Fläche dient als Unterseitenfläche 4 und hat in etwa die Form einer halben Ellipse. Dabei stellt eine Ellipsennebenachse eine Grundseite der Unterseitenfläche 4 dar, wobei diese gebogen ist, derart, dass sie die Unterseitenfläche 4 verglichen mit einer geraden Ellipsennebenachse verkleinert.

Ebenso wäre es denkbar, dass sich die Grundseite in eine entgegengerichtete Richtung erstreckt, derart, dass sich die Unterseitenfläche 4 vergrößert.

An der Ellipsennebenachse der Unterseitenfläche 4 schließt sich über eine verrundete Kante ein Lagerabschnitt bzw. eine Lagerfläche 6 an, der im Wesentlichen in Einführrichtung senkrecht zur Fläche 4 angeordnet ist. An der anderen Kante der Unterseitenfläche 4 schließt sich über eine verrundete Kante eine als Rückseite des Heart Pad 1 dienende Stützfläche 2 bzw. Auflagefläche an, mit welcher sich das Heart Pad 1 an einer Umgebung abstützt. Die Stützfläche 2 ist als verrundete, konvexe Außenmantelfläche ausgeführt und hat einen größten Querschnittsdurchmesser auf der Seite der Unterseitenfläche 4 hin, wohingegen der Querschnittsdurchmesser zum anderen Ende hin (in Einführrichtung) abnimmt. Mit anderen Worten, entspricht die Stützfläche 2 im Wesentlichen einem in Längsrichtung vorderen Endabschnitt einer verrundeten Wanne.

Als Montage- und Entnahmehilfe 8 dient ein als eine Öse oder Schlaufe ausgebildetes Element, zu dem sich die Stützfläche 2 abschnittsweise am Scheitel der Halbellipse erweitert und in etwa lotrecht zur Unterseitenfläche 4 vorspringt. Die Montage- und Entnahmehilfe 8 kann gleichermaßen in Form einer Vertiefung in der Außenhaut des Heart Pad 1 ausgebildet sein, an der ein Steg ausgebildet ist, an dem das Heart Pad 1 gegriffen werden kann.

Wie in Fig. 1 zu erkennen ist, hat das Heart Pad 1 an seiner Unterseite vergleichsweise sowohl in der Breite als auch in der Tiefe die größten Ausmaße, wohingegen es sich in Richtung der Oberseite, mit welcher das Heart Pad 1 voran in einen Herzbeutel eingeführt wird, verjüngt. Dadurch wird eine im Wesentlichen keilförmige Ausgestaltung realisiert, so dass das Heart Pad 1 durch den in Einführrichtung zusammenlaufenden vorderen Bereich einfacher in die Perikardhöhle eingeführt werden kann. Zugleich kann das Heart Pad 1 durch den sperrigeren in Einführrichtung hinteren Bereich besser zwischen dem Perikard und dem Epikard geklemmt werden.

Bevorzugter Weise wird das Heart Pad 1 mit einem in jedem Siebkorb befindlichen Instrument wie einer Zange oder dergleichen (nicht dargestellt) über die Montage- und Entnahmehilfe 8 geführt.

Fig. 2 ist eine perspektivische Darstellung des Heart Pads 1 von Fig. 1, in der eine Vorderseite des Heart Pads 1 zu sehen ist. Die Vorderseite weist einen Lagerabschnitt 6 auf, der als verrundete, konkave Fläche ausgebildet ist. Der Lagerabschnitt 6 ist einer Oberfläche des Herzen formstabil nachempfunden und bildete folglich eine Negativform zur Herzoberfläche. Folglich kann das Herz (nicht dargestellt) über den optimal an die Herzkontur angepassten Lagerabschnitt 6 gestützt und gehalten werden. Im Idealfall stellen der Lagerabschnitt 6 und die Außenhaut des Herzen zwei korrespondieren Flächen dar, die nahezu perfekt zueinander passen.

Des Weiteren ist der Lagerabschnitt 6 an Randbereichen mit Aussparungen 10 entlang der jeweiligen Kante zwischen der Lagerabschnitt 6 und der Stützfläche 2 versehen. Diese sind vorzugsweise an den Stellen angebracht, an denen im eingeführten Zustand des Heart Pads 1 wichtige bzw. ausgeprägte Gefäße und / oder Gefäßstrukturen vorhanden sind. Entsprechend dem jeweiligen Gefäß oder der jeweiligen Gefäßstruktur, welche von der muldenförmigen Aussparung 10 aufgenommen wird, weist sie einen größeren, kleineren oder ungleichmäßigen Radius auf. Dabei können sowohl eine Tiefe, als auch eine Breite sowie eine Anzahl von Aussparungen 10 variiert werden.

Die keilförmige Ausprägung des Heart Pads 1 findet sich auch im Lagerabschnitt 6 wieder. Dieser ist am einen Endabschnitt auf der Seite der Unterseitenfläche 4 am breitesten und wird in Einführrichtung des Heart Pads 1 immer schmäler. Am anderen Endabschnitt des Lagerabschnitts 6 geht dieser in einen Lagerflächenvorderabschnitt 12 über, welcher eine Keilspitze darstellt. Diese kann symmetrisch oder asymmetrisch ausgebildet sein. Ferner kann die Keilspitze 12 in Längsrichtung zum Lagerabschnitt 6 ebenso mittig wie desachsiert sein, so dass das Heart Pad 1 möglichst widerstandsarm in die Perikardhöhle eingeführt und gegebenenfalls zwischen Gefäßen vorbeigeschoben und platziert werden kann.

Ferner weist das Heart Pad 1 auf der Fläche des Lagerabschnitts 6 eine Kennzeichnung 16 auf. Diese trägt eine Beschriftung sowie einen Pfeil, so dass der Operateur eindeutig erkennen kann, mit welcher Orientierung das Heart Pad 1 einzulegen ist. Wie in Fig. 2 dargestellt, kann die Beschriftung konkrete Gefäße wie beispielsweise die Vena cava inferior (untere Hohlvene) bezeichnen. Sie kann ebenso andere Hinweise aufweisen, wie etwa eine Orientierung des Heart Pads 1 zum Körper des Patienten.

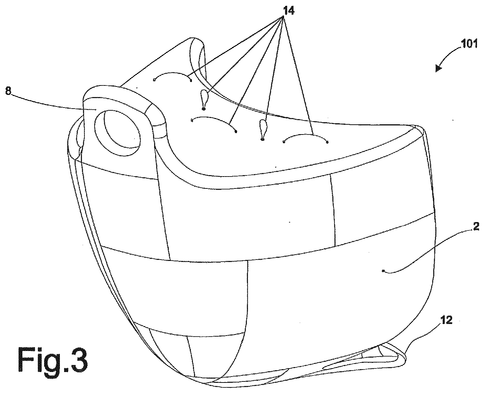

In Fig. 3 ist eine schematische, perspektivische Darstellung eines Heart Pads 101 in einer Rückansicht gemäß einem zweiten Ausführungsbeispiel der Erfindung gezeigt. Das zweite Ausführungsbeispiel unterscheidet sich vom ersten Ausführungsbeispiel dahingehend, als dass es zusätzlich zumindest eine, vorzugsweise mehrere an der Unterseitenfläche 40 angebrachte Hilfs- und Haltevorrichtungen 14 aufweist. Die als Schlaufen 14 ausgebildeten Hilfs- und Haltevorrichtungen in in etwa regelmäßigen Abständen eignen sich besonders zum Anbringen von Hilfs- und Haltenähten, die während der Operation vorübergehend benötigt werden. Die Schlaufen 14 können ebenso an anderen Flächen des Heart Pads vorgesehen sein.

Ferner können anstelle von Schlaufen ebenso mit Ösen versehene Laschen (nicht dargestellt) verwendet werden. Diese haben den zusätzlichen Vorteil, dass sie bündig an der jeweiligen Fläche anliegen, und bei Bedarf hochgeklappt werden können. Alternativ oder ergänzend können muldenförmige Vertiefungen in der Oberfläche des Heart Pads vorgesehen sein, bei denen zumindest ein Steg vorhanden ist, woran Nähte befestigt werden können.

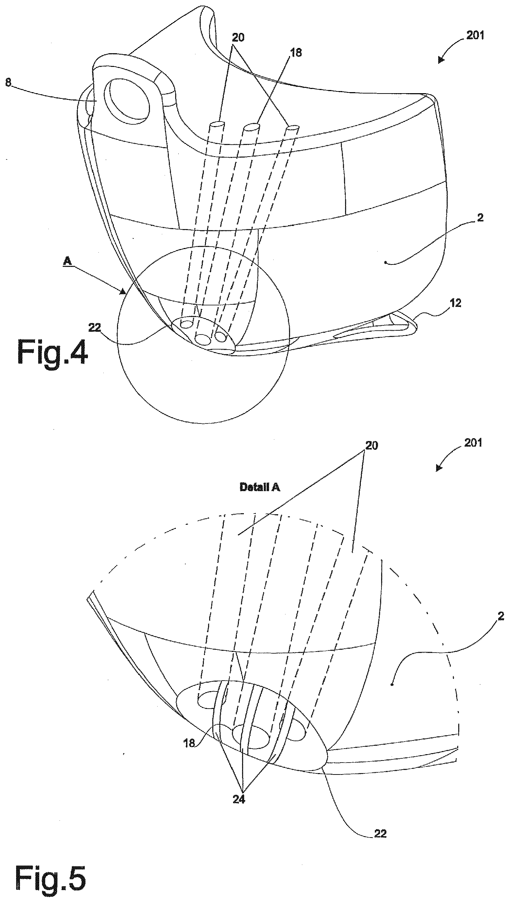

Fig. 4 zeigt eine schematische, perspektivische Darstellung eines Heart Pads 201 in einer Rückansicht gemäß einem dritten Ausführungsbeispiel der Erfindung. Mittels der transparenten Darstellung des Heart Pads 201 sind darin verlaufende Kanäle 18, 20 erkennbar. Zumindest ein Kanal dient als Saugkanal bzw. Absaugkanal 18 zum Abführen von freien Flüssigkeiten aus dem Operationsbereich heraus. Der Absaugkanal 18 hat an seinen beiden Enden jeweils eine kreisrunde Öffnung, von denen sich die proximale Öffnung an der Unterseitenfläche 4, und die distale Öffnung an einer Aussparung bzw. Saugöffnung 22 befindet. Die Saugöffnung 22 ist in der Stützfläche 2 in dem Bereich des Heart Pads 201 eingebracht, der sich im eingeführten Zustand beim liegenden Patienten an der im Wesentlichen untersten und am weitesten eingeführten Stelle befindet. Auf diese Weise können am für den Operateur am schwierigsten erreichbaren Bereich sich sammelnde Flüssigkeiten abgeführt werden.

Neben dem Absaugkanal 18 ist zumindest ein weiterer Kanal 20 vorgesehen, der als Luftkanal 20 zum Nachströmen von Luft dient. Der Luftkanal 20 verläuft in etwa parallel zum Absaugkanal 18. Ein proximales Ende befindet sich an der Unterseitenfläche 4, während sich das distale Ende an der Saugöffnung 22 möglichst nahe der distalen Öffnung des Absaugkanals 18 befindet. Damit ausreichend Luft entsprechend dem abgesaugten Volumen der Flüssigkeit durch den Absaugkanal 18 nachströmt, sind vorzugsweise zwei Luftkanäle 20 vorgesehen, wobei diese jeweils in etwa den gleichen Durchmesser wie der Absaugkanal 18 aufweisen. Auf diese Weise kann verhindert werden, dass im Absaugbereich ein relativer Unterdruck erzeugt wird, infolgedessen das umliegende Gewebe angesaugt wird und womöglich die Saugöffnung 22 verdeckt.

Der Absaugkanal 18 kann wahlweise an seinem proximalen Ende an eine Handabsaugpumpe, eine manuelle Absaugpumpe (Fußbetrieb) und / oder eine elektrisch betriebene Absaugpumpe bzw. einen Sauger angeschlossen werden. Um sicherzustellen, dass nicht eine Flüssigkeit anstelle von Luft durch die Luftkanäle nachströmt, können auch diese an ihren proximalen Enden an ein beliebiges Entlüftungssystem angeschlossen werden. Als Verbindungssystem kommt vorzugsweise das vereinheitlichte Luer-Lock (nicht dargestellt) zum Einsatz.

Anstatt einer externen Saugvorrichtung könnte eine in das Heart Pad 1 integrierte interne Saugvorrichtung verwendet werden.

Fig. 5 zeigt eine Detailansicht der Saugöffnung 22 in Fig. 4. Hier ist gezeigt, dass die Kanäle 18 und 20 etwas nach innen in das Heart Pad versetzt sind, um nicht über die Außenfläche der Stützfläche 2 hervorzuragen. Damit wird ein Ansaugen des umliegenden Gewebes verhindert. Ferner wird für ein optimales Nachströmen von Luft der Absaugkanal 18 teilweise von den Luftkanälen 20 umgeben, wobei deren jeweiligen distalen Enden möglichst nah aneinander anzuordnen sind.

Zudem sind Streben 24 vorgesehen, die diametral über der Saugöffnung 22 angeordnet sind und verhindern, dass das Gewebe von der distalen Öffnung des Absaugkanals 18 angesaugt wird und folglich diese versperrt. Die Streben 24 sind bevorzugt leicht gebogen ausgebildet, so dass sie das Gewebe zusätzlich von der Saugöffnung 22 beabstanden.

Obwohl in Fig. 5 drei im Wesentlichen vertikal verlaufende Streben 24 gezeigt sind, ist die Erfindung nicht hierauf beschränkt. Daher können ebenso mehr oder weniger Streben verwendet werden, deren Orientierung beliebig gewählt werden kann. Außerdem kann eine Gitterstruktur verwendet werden. Bei der Wahl der Schutzvorrichtung ist zu beachten, dass Flüssigkeiten abgeführt werden können. Dazu ist wichtig, dass zum einen die Öffnung des Absaugkanals 18 nicht versperrt wird, und zum anderen, dass ausreichend Luft nachströmt.

Abweichend von den oben beschriebenen Ausführungsbeispielen kann das Heart Pad vollständig oder teilweise, vorzugweise an der Unterseitenfläche 4 teilweise oder vollständig mit einem Stoff (nicht dargestellt) in Form eines Netzes oder Textilüberzugs versehen sein. Daran können auf einfache Weise Hilfs- und Haltenähte angebracht werden. Kommt ein Kunststoff mit geringer Weiterreißfestigkeit zum Einsatz, kann ein Ausreißen von Nähten verhindert werden.

Außerdem kann ein Stoffüberzug ebenso am Lagerabschnitt bzw. am Stützabschnitt vorgesehen sein, wodurch ein Verrutschen unterdrückt und die Positionierung des Herzen verbessert wird. Die gleiche Wirkung kann durch eine entsprechende Oberflächenbeschaffenheit erreicht werden. Sie kann eine erhöhte Rauhigkeit und / oder entsprechend orientierte Schuppen aufweisen.

Ebenso sind Kombinationen der beschriebenen Ausführungsbeispiele denkbar. So kann das Heart Pad 201 gemäß dem dritten Ausführungsbeispiel sämtliche Merkmale der anderen Ausführungsbeispiele aufweisen.

Als Werkstoff kommt bevorzugt Kunststoff in Frage, wobei ebenso ein metallischer Werkstoff oder Keramiken einsetzbar sind. Besonders bevorzugt ist ein wärmedämmendes Material, das ein zu starkes Abkühlen verhindert.

Zusammenfassend betrifft der Kern der Erfindung ein chirurgisches Instrument zum Positionieren von Organen, insbesondere von Herzen, das die Hämodynamik des Herzen sowie die Funktion des jeweiligen Organs nicht einschränkt und zugleich dem Operateur eine gute Sicht auf das Organ sicherstellt. Dabei wird besonders den vergleichbaren bekannten Lösungen Rechnung getragen, die entweder sehr sperrig und unhandlich bzw. kostspielig sind und keine Rücksicht auf die Form der Organe nehmen. Das Heart Pad der vorliegenden Erfindung positioniert auf kostengünstige Weise insbesondere mittels der idealen Passform das Herz mir geringem Montageaufwand, während es die Hämodynamik des Herzen nicht einschränkt. Da das Heart Pad in die Perikardhöhle gelegt wird und das Herz individuell und in beliebiger Position stützt, können Operationen an der Herzrückwand bzw. an Herzseitenwänden durchgeführt werden. Es sein an dieser Stelle betont, dass die Auflageflächen derart an das Herz und dessen Umgebung angepasst sind, dass alle vorkommenden Herzgrößen möglichst ideal positioniert werden können.

### Bezugszeichenliste

- 1, 101, 201: Stützkörper / Heart Pad
- 2: Stützfläche
- 4: Unterseitenfläche
- 6: Lagerabschnitt bzw. Lagerfläche
- 8: Montage- und Entnahmehilfe
- 10: Aussparung
- 12: Keilspitze
- 14: Hilfs- und / oder Haltevorrichtung bzw. Schlaufen
- 16: Kennzeichnung
- 18: Kanal bzw. Absaugkanal
- 20: Luftkanal bzw. Entlüftungskanal
- 22: Aussparung bzw. Saugöffnung
- 24: Streben

## Patentansprüche

1. Chirurgisches Organ-Positionierinstrument zum Abstützen und Halten von Organen während einer Operation, wobei das Organ-Positionierinstrument einen Stützkörper (1, 101, 201) aufweist mit:
zwei Außenecken,
einer ersten gebogenen verrundeten Kante, die von einer Außenecke zur anderen Außenecke verläuft, und die eine im Wesentlichen plane Unterseitenfläche (4) an eine konkave verrundete Lagerfläche (6) anschließt,
einer zweiten konvexen Kante, die von einer Außenecke zur anderen Außenecke verläuft, und die die Lagerfläche (6) an eine konvexe verrundete Stützfläche (2) anschließt, und
einer dritten konvexen verrundeten Kante, die von einer Außenecke zur anderen Außenecke verläuft, und die die Stützfläche (2) an die Unterseitenfläche (4) anschließt, wobei die Lagerfläche (6) an einem Endabschnitt auf der Seite der Unterseitenfläche (4) am breitesten ist und in Richtung ihres anderen Endabschnitts schmäler wird und schließlich in einen Lagerflächenvorderabschnitt (12) übergeht, welcher eine Keilspitze (12) darstellt,
**dadurch gekennzeichnet, dass**
die dritte Kante im Wesentlichen die Form einer halben Ellipse aufweist, deren Ellipsennebenachse von einer Außenecke zur anderen Außenecke des Stützkörpers (1, 101, 201) verläuft,
die Lagerfläche (6) im Wesentlichen senkrecht zu der Unterseitenfläche (4) angeordnet ist, und
sich der Stützkörper (1, 101, 201) ausgehend von der Unterseitenfläche (4) in Richtung des Lagerflächenvorderabschnitts (12) in allen zur Unterseitenfläche (4) parallelen Dimensionen verjüngt.

2. Chirurgisches Organ-Positionierinstrument nach Anspruch 1, **dadurch gekennzeichnet, dass** der Stützkörper (1, 101; 201) eine integrierte Sensor-/Aktuatorkombination aufweist, die zum Ruhigstellen einer Operationsstelle mit einem elektrischen Strom beaufschlagbar ist.

3. Chirurgisches Organ-Positionierinstrument nach Anspruch 1 oder 2, **dadurch gekennzeichnet, dass** das der Stützkörper (1, 101, 201) im Bereich der Lagerfläche (6) mit wenigstens einer zusätzlichen Auskerbung oder Einbuchtung (10) an dessen Oberfläche versehen ist, die ihrerseits eine Auflagerfläche ausbildet, welche zur großflächigen Gewebeauflagerung vorzugsweise von an das Ziel-Organ angeschlossenen Körperteilen angepasst ist, wobei die wenigstens eine zusätzliche Auskerbung oder Einbuchtung (10) an die Lagerfläche (6) in diese erweiternder Weise angrenzt und ebenfalls an anatomischen Gegebenheiten des aufgelagerten Gewebes angeglichen ist.

4. Chirurgisches Organ-Positionierinstrument nach Anspruch 3, **dadurch gekennzeichnet, dass** die wenigstens eine zusätzliche Auskerbung oder Einbuchtung (10) im Winkel zur Lagerfläche (6) ausgerichtet ist, derart, dass sich eine Kante zwischen der Lagerfläche (6) und der Auskerbung oder Einbuchtung (10) ergibt.

5. Chirurgisches Organ-Positionierinstrument nach einem der Ansprüche 1 bis 4, **dadurch gekennzeichnet, dass** der Stützkörper (1, 101, 201) ferner einen vorzugsweise als Öse oder Schlaufe ausgebildeten Greifabschnitt (8) aufweist, über welchen der Stützkörper (1, 101, 201) über ein chirurgisches Instrument anbringbar und / oder entfernbar ist.

6. Chirurgisches Organ-Positionierinstrument nach einem der Ansprüche 1 bis 5, **dadurch gekennzeichnet, dass** der Stützkörper (1, 101, 201) eine Hilfs- und / oder Haltevorrichtung (14) aufweist, an welcher vorzugsweise Hilfsnähte anbringbar sind.

7. Chirurgisches Organ-Positionierinstrument nach einem der Ansprüche 1 bis 6, **dadurch gekennzeichnet, dass** der Stützkörper (1, 101, 201) eine Abführvorrichtung mit zumindest einem als Ausleitkanal und / oder Luftnachführkanal ausgebildeten Kanal aufweist, wobei
ein erstes Ende des Kanals innerhalb der Lagerfläche (6) zur Umgebung mündet und dessen anderes Ende innerhalb der konvex verrundeten Stützfläche (2) zur Umgebung mündet, wobei eine Saugeinrichtung vorgesehen ist, die an den zumindest einen Kanal an der Mündung in der konvex verrundeten Stützfläche (2) anschließbar ist.

8. Chirurgisches Organ-Positionierinstrument nach einem der Ansprüche 1 bis 6, **dadurch gekennzeichnet, dass**
der Stützkörper (201) eine Abführvorrichtung mit zumindest einem als Ausleitkanal ausgebildeten Kanal (18) und mit zumindest einem als Luftnachführkanal ausgebildeten Luftkanal (20) aufweist, wobei
erste Enden der Kanäle (18, 20) innerhalb der Lagerfläche (6) zur Umgebung münden und andere Enden innerhalb der konvex verrundeten Stützfläche (2) zur Umgebung münden, wobei eine Saugeinrichtung vorgesehen ist, die an den Ausleitkanal (18) an der Mündung in der konvex verrundeten Stützfläche (2) anschließbar ist.

9. Chirurgisches Organ- Positionierinstrument nach einem der Ansprüche 1 bis 8, **dadurch gekennzeichnet, dass** der Stützkörper zumindest an der Lagerfläche (6) und/oder an der zusätzlichen Auskerbung oder Einbuchtung (10) ein wärmedämmendes Material und / oder ein Thermoelement aufweist, wobei unter wärmedämmend in bevorzugter Weise ein Material, vorzugsweise ein elastisches Material verstanden werden soll mit einer Wärmeleitfähigkeit von maximal 1W/(m*K).

10. Chirurgisches Organ-Positionierinstrument nach einem der Ansprüche 1 bis 9, **dadurch gekennzeichnet, dass** der Stützkörper eine auf Unterdruck basierende Fixiervorrichtung zwischen der Lagerfläche (6) und dem zu stützenden Ziel-Organ und / oder zwischen dem rückseitigen Stützabschnitt und der Umgebung aufweist.

11. Chirurgisches Organ-Positionierinstrument nach einem der Ansprüche 1 bis 9, **dadurch gekennzeichnet, dass** zumindest ein Teilbereich der Lagerfläche (6) mit einem Netz und / oder Textilüberzug versehen ist.

12. Chirurgisches Organ-Positionierinstrument nach einem der Ansprüche 1 bis 11, **dadurch gekennzeichnet, dass** der Stützkörper eine Vielzahl von individuell befüllbaren Kammern aufweist.

13. Chirurgisches Organ-Positionierinstrument nach einem der Ansprüche 1 bis 12, **dadurch gekennzeichnet, dass** der Stützkörper eine Kombination aus einem weichen, vorzugsweise die äußere Hülle betreffenden, und einem der gegenüber steifen, vorzugsweise den Kern betreffenden Bereich ist.

14. Chirurgisches Organ-Positionierinstrument nach einem der Ansprüche 1 bis 13, **dadurch gekennzeichnet, dass** der Stützkörper eine Hülle aufweist, die zumindest im Bereich der Lagerfläche (6) und / oder der Stützfläche (2) mit einem Granulat befüllt ist, wobei der Hülle Luft entzogen werden kann.

15. Chirurgisches Organ-Positionierinstrument nach einem der Ansprüche 1 bis 14, **dadurch gekennzeichnet, dass** das Instrument ein modular aufgebautes System ist.

## Claims

1. A surgical organ positioning instrument for supporting and holding organs during surgery, wherein the organ positioning instrument comprises a supporting portion (1, 101, 201) having:
two outer corners,
a first curved, rounded edge extending from one outer corner to the other outer corner and connecting a substantially planar underside surface (4) to a concave, rounded bearing surface (6)
a second convex edge extending from one outer corner to the other outer corner and connecting the bearing surface (6) to a convex, rounded supporting surface (2), and
a third convex, rounded edge extending from one outer corner to the other outer corner and connecting the supporting surface (2) to the underside surface (4),
wherein the bearing surface (6) is widest at one end portion on the side of the underside surface (4) and narrows toward its other end portion and finally merges into a bearing surface front portion (12) which constitutes a wedge tip (12),
**characterized in that**
the third edge is substantially in the form of a half ellipse, the ellipse minor axis of which extends from one outer corner to the other outer corner of the supporting portion (1, 101, 201),
the bearing surface (6) is arranged substantially perpendicular to the underside surface (4), and
the supporting portion (1, 101, 201) tapers from the underside surface (4) in the direction of the bearing surface front portion (12) in all dimensions parallel to the underside surface (4).

2. The surgical organ positioning instrument according to claim 1, **characterized in that** the supporting portion (1; 101; 201) includes an integrated sensor-actuator combination to which electricity can be applied for immobilizing a site of operation.

3. The surgical organ positioning instrument according to claim 1 or 2, **characterized in that** in the area of the bearing surface (6) the supporting portion (1; 101; 201) is provided with at least one further recess or indentation (10) at the surface thereof forming a support face adapted for large-scale tissue support preferentially of body parts connected to the target organ, wherein the at least one additional recess or indentation (10) is adjacent to the bearing surface (6) in a way expanding the latter and is also adapted to the anatomic conditions of the supported tissue.

4. The surgical organ positioning instrument according to claim 3, **characterized in that** the at least one additional recess or indentation (10) is oriented at an angle to the bearing surface (6), so that a ridge is formed between the bearing surface (6) and the recess or indentation (10).

5. The surgical organ positioning instrument according to one of the claims 1 to 4, **characterized in that** the supporting portion (1; 101; 201) further includes a gripping portion (8) preferably in the form of an eye or a loop by which the supporting portion (1; 101; 201) can be arranged and/or removed via a surgical instrument.

6. The surgical organ positioning instrument according to one of the claims 1 to 5, **characterized in that** the supporting portion (1; 101; 201) includes an auxiliary and/or holding device (14) to which preferably auxiliary sutures can be attached.

7. The surgical organ positioning instrument according to one of the claims 1 to 6, **characterized in that** the supporting portion (1; 101; 201) includes a discharge device including at least one passage in the form of a drain passage and/or an air resupply passage, wherein
a first end of the passage opens to the environment in the bearing surface (6), and the other end of the passage opens to the environment in the convexly rounded supporting surface (2), wherein suction means are provided that are connectable to the at least one passage at the opening in the convexly rounded supporting surface (2).

8. The surgical organ positioning instrument according to one of the claims 1 to 6, **characterized in that**
the supporting portion (201) includes a discharge device comprising at least one passage (18) in the form of a drain passage and at least one air passage (20) in the form of an air resupply passage, wherein
first ends of the passages (18, 20) open to the environment in the bearing surface (6), and other ends open to the environment in the convexly rounded supporting surface (2), wherein suction means are provided that are connectable to the drain passage (18) at the opening in the convexly rounded supporting surface (2).

9. The surgical organ positioning instrument according to one of the claims 1 to 8, **characterized in that** the supporting portion includes a heat-insulating material and/or a thermal element at least at the bearing surface (6) and /or at the additional recess or indentation (10), wherein the heat-insulating material preferentially is understood to be an elastic material with a heat conductivity of maximally 1W/(m*K).

10. The surgical organ positioning instrument according to one of the claims 1 to 9, **characterized in that** the supporting portion includes a vacuum-based fixation means between the bearing surface (6) and the target organ to be supported and/or between the rearward supporting portion and the environment.

11. The surgical organ positioning instrument according to one of the claims 1 to 9, **characterized in that** at least a surface area fraction of the bearing surface (6) is provided with a mesh and/or textile coating.

12. The surgical organ positioning instrument according to one of the claims 1 to 11, **characterized in that** the supporting portion includes a plurality of chambers which can be individually filled.

13. The surgical organ positioning instrument according to one of the claims 1 to 12, **characterized in that** the supporting portion is a combination of a soft portion preferably relating to the outer shell and a rigid portion preferably relating to the core.

14. The surgical organ positioning instrument according to one of the claims 1 to 13, **characterized in that** the supporting portion includes a shell which is filled with granulate material at least in the area of the bearing surface (6) and/or at the supporting surface (2), wherein air can be withdrawn from the shell.

15. The surgical organ positioning instrument according to one of the claims 1 to 14, **characterized in that** the positioning instrument is a modular system.

## Revendications

1. Instrument chirurgical de positionnement d'organes pour soutenir et retenir des organes pendant une opération, dans lequel l'instrument de positionnement d'organes présente un corps de soutien (1, 101, 201) avec :
deux coins extérieurs,
une première arête arrondie courbée qui s'étend d'un coin extérieur à l'autre coin extérieur, et qui raccorde une surface latérale inférieure sensiblement plane (4) à une surface de support arrondie concave (6),
une deuxième arête convexe qui s'étend d'un coin extérieur à l'autre coin extérieur, et qui raccorde la surface de support (6) à une surface de soutien arrondie convexe (2), et
une troisième arête arrondie convexe qui s'étend d'un coin extérieur à l'autre coin extérieur, et qui raccorde la surface de soutien (2) à la surface latérale inférieure (4),
dans lequel la surface de support (6) est la plus large au niveau d'une section d'extrémité sur le côté de la surface latérale inférieure (4) et finalement passe dans une section avant de surface de support (12), laquelle représente une pointe de coin (12),
**caractérisé en ce que**
la troisième arête présente sensiblement la forme d'une demi-ellipse, dont l'axe secondaire d'ellipse s'étend d'un coin extérieur à l'autre coin extérieur du corps de soutien (1, 101, 201),
la surface de support (6) est disposée sensiblement perpendiculairement à la surface latérale inférieure (4), et
le corps de soutien (1, 101, 201) se rétrécit en partant de la surface latérale inférieure (4) dans la direction de la section avant de surface de support (12) dans toutes les dimensions parallèles à la surface latérale inférieure (4).

2. Instrument chirurgical de positionnement d'organes selon la revendication 1, **caractérisé en ce que** le corps de soutien (1, 101 ; 201) présente une combinaison capteur/actionneur intégrée qui peut être sollicitée pour l'immobilisation d'une position d'opération avec un courant électrique.

3. Instrument chirurgical de positionnement d'organes selon la revendication 1 ou 2, **caractérisé en ce que** le corps de soutien (1, 101, 201) est pourvu dans la zone de la surface de support (6) d'au moins une encoche ou échancrure (10) supplémentaire sur la surface de celui-ci qui réalise de son côté une surface portante qui est adaptée à une pose de tissu large, de préférence de parties de corps raccordées à l'organe cible, dans lequel l'au moins une encoche ou échancrure (10) supplémentaire est adjacente à la surface de support (6) de cette manière élargissante et est également ajustée à des réalités anatomiques du tissu posé.

4. Instrument chirurgical de positionnement d'organes selon la revendication 3, **caractérisé en ce que** l'au moins une encoche ou échancrure (10) supplémentaire est orientée selon un angle par rapport à la surface de support (6) de telle sorte qu'il en résulte une arête entre la surface de support (6) et l'encoche ou échancrure (10).

5. Instrument chirurgical de positionnement d'organes selon l'une des revendications 1 à 4, **caractérisé en ce que** le corps de soutien (1, 101, 201) présente en outre une section de préhension (8) réalisée de préférence en tant qu'œillet ou passant, par le biais de laquelle le corps de soutien (1, 101, 201) peut être installé et/ou retiré par le biais d'un instrument chirurgical.

6. Instrument chirurgical de positionnement d'organes selon l'une des revendications 1 à 5, **caractérisé en ce que** le corps de soutien (1, 101, 201) présente un dispositif auxiliaire et/ou de maintien (14) au niveau duquel une jointure auxiliaire peut être installée.

7. Instrument chirurgical de positionnement d'organes selon l'une des revendications 1 à 6, **caractérisé en ce que** le corps de soutien (1, 101, 201) présente un dispositif d'évacuation avec au moins un canal réalisé en tant que canal de dérivation et/ou canal de suivi d'air, dans lequel
une première extrémité du canal débouche à l'intérieur de la surface de support (6) dans l'environnement et dont un autre extrémité débouche à l'intérieur de la surface de soutien arrondie de manière convexe (2) dans l'environnement, dans lequel un dispositif d'aspiration est prévu, qui peut être raccordé à l'au moins un canal au niveau de l'embouchure dans la surface de soutien arrondie de manière convexe (2).

8. Instrument chirurgical de positionnement d'organes selon l'une des revendications 1 à 6, **caractérisé en ce que**
le support (201) présente un dispositif d'évacuation avec au moins un canal (18) réalisé en tant que canal de dérivation et avec au moins un canal d'air (20) réalisé en tant que canal de suivi d'air, dans lequel
des premières extrémités des canaux (18, 20) débouchent à l'intérieur de la surface de support (6) dans l'environnement et d'autres extrémités débouchent à l'intérieur de la surface de soutien arrondie de manière convexe (2) dans l'environnement, dans lequel un dispositif d'aspiration est prévu, qui peut être raccordée au canal de dérivation (18) au niveau de l'embouchure dans la surface de soutien arrondie de manière convexe (2).

9. Instrument chirurgical de positionnement d'organes selon l'une des revendications 1 à 8, **caractérisé en ce que** le support présente au moins au niveau de la surface de support (6) et/ou au niveau de l'encoche ou échancrure (10) supplémentaire un matériau isolant et/ou un élément thermique, dans lequel il faut comprendre par isolant de préférence un matériau, de préférence un matériau élastique, avec une capacité caloporteuse de 1 W/(m^{∗}K) au maximum.

10. Instrument chirurgical de positionnement d'organes selon l'une des revendications 1 à 9, **caractérisé en ce que** le support présente un dispositif de fixation se basant sur une dépression entre la surface de support (6) et l'organe cible à soutenir et/ou entre la section de soutien arrière et l'environnement.

11. Instrument chirurgical de positionnement d'organes selon l'une des revendications 1 à 9, **caractérisé en ce qu'au** moins une partie de la surface de support (6) est pourvue d'un maillage et/ou d'un revêtement textile.

12. Instrument chirurgical de positionnement d'organes selon l'une des revendications 1 à 11, **caractérisé en ce que** le support présente une pluralité de chambres aptes à être remplies individuellement.

13. Instrument chirurgical de positionnement d'organes selon l'une des revendications 1 à 12, **caractérisé en ce que** le support est une combinaison d'une zone molle, de préférence concernant l'enveloppe extérieure, et d'une zone rigide opposée, concernant de préférence le cœur.

14. Instrument chirurgical de positionnement d'organes selon l'une des revendications 1 à 13, **caractérisé en ce que** le support présente une enveloppe qui est remplie d'un granulat au moins dans la zone de la surface de support (6) et/ou de la surface de soutien (2), dans lequel de l'air peut être retiré de l'enveloppe.

15. Instrument chirurgical de positionnement d'organes selon l'une des revendications 1 à 14, **caractérisé en ce que** l'instrument est un système modulaire.
